(19) **Europäisches Patentamt / European Patent Office / Office européen des brevets**

(11) **EP 4 394 352 B1**

(12) # FASCICULE DE BREVET EUROPEEN

(45) Date de publication et mention
de la délivrance du brevet:
**01.07.2026  Bulletin 2026/27**

(21) Numéro de dépôt: **23220208.5**

(22) Date de dépôt: **26.12.2023**

(51) Classification Internationale des Brevets (IPC):
**G01N 15/1429** *(2024.01)*   **G01N 21/64** *(2006.01)*
**G01N 15/1433** *(2024.01)*   **C12N 5/071** *(2010.01)*
**G01N 15/10** *(2024.01)*   **G01N 15/1434** *(2024.01)*

(52) Classification Coopérative des Brevets (CPC):
**G01N 15/1429; C12N 5/0612; G01N 15/1433;**
**G01N 21/6458;** G01N 2015/1006; G01N 2015/1454;
G01N 2021/6419; G01N 2021/6421

(54) **DISPOSITIF ET PROCÉDÉ D'OBSERVATION D'UNE FLUORESCENCE OU D'UNE LUMINESCENCE D'UNE PARTICULE MOBILE**

VORRICHTUNG UND VERFAHREN ZUR BEOBACHTUNG EINER FLUORESZENZ ODER EINER LUMINESZENZ EINES BEWEGLICHEN TEILCHENS

DEVICE AND METHOD FOR OBSERVING FLUORESCENCE OR LUMINESCENCE OF A MOVING PARTICLE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité:  **27.12.2022  FR 2214523**

(43) Date de publication de la demande:
**03.07.2024  Bulletin 2024/27**

(73) Titulaire: **Commissariat à l'Energie Atomique et aux Energies Alternatives**
**75015 Paris (FR)**

(72) Inventeur: **MANDULA, Ondrej**
**38054 Grenoble cedex 09 (FR)**

(74) Mandataire: **INNOV-GROUP**
**209 Avenue Berthelot**
**69007 Lyon (FR)**

(56) Documents cités:
WO-A1-2017/214023     US-A1- 2016 169 801
US-A1- 2018 293 426     US-B1- 6 259 807

## Description

## DOMAINE TECHNIQUE

Le domaine technique de l'invention concerne d'observation d'une fluorescence ou d'une luminescence d'une particule mobile.

## ART ANTERIEUR

Le recours à l'imagerie de fluorescence est une technique bien connue pour caractériser des particules, en particulier dans le domaine de la biologie. Cependant, la lumière de fluorescence est souvent de faible intensité. Une pratique courante consiste à acquérir une image de fluorescence selon une grande durée d'acquisition, ou de cumuler différentes images de fluorescence. Cela permet d'obtenir une image de fluorescence dont le rapport signal sur bruit est acceptable.

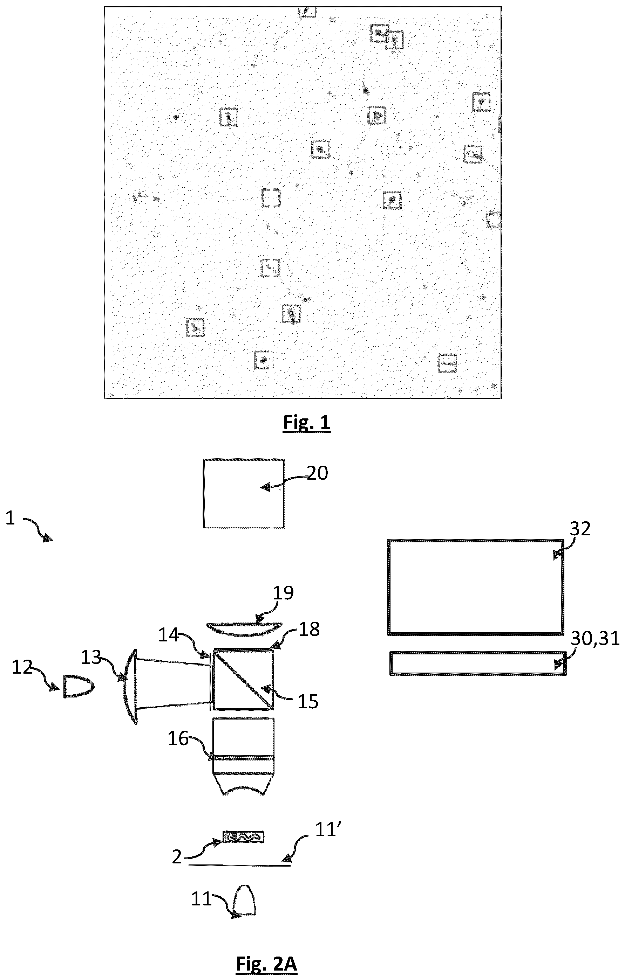

La figure 1 représente un échantillon comportant des spermatozoïdes, ces derniers étant mobiles. Sur de telles particules, les pratiques usuelles consistent à augmenter la durée d'acquisition, ou de cumuler différentes images, ne sont pas adaptées. En effet, les particules bougent selon une trajectoire difficilement prévisible. Et chaque particule suit une trajectoire différente d'une autre particule.

Un dispositif permettant d'examiner la fluorescence de particules a été décrit dans US20180293426. Dans ce dispositif, des particules sont déplacées devant un microscope. Des images des particules sont formées selon différentes modalités. Les particules ont toutes le même mouvement, car il s'agit d'un déplacement d'ensemble de l'échantillon par rapport au microscope. Un tel dispositif n'est pas adapté à des particules dont les déplacements, dans l'échantillon, sont aléatoires.

WO2017214023 décrit une acquisition d'images de cellules selon une modalité multispectrale, à l'aide d'un dispositif de type cytomètre en flux. US2016169801 décrit un dispositif permettant une acquisition simultanée de différentes images de particules, selon différentes bandes spectrales de fluorescence. US6259807 décrit une formation d'images de particules selon différentes modalités de particules non mobiles dans un échantillon.

L'invention décrite ci-après permet de surmonter cette difficulté. Elle rend possible l'observation de la fluorescence de particules mobiles. L'invention s'applique également à l'observation de la luminescence d'une particule mobile.

## EXPOSE DE L'INVENTION

Un premier objet de l'invention est un procédé d'observation d'une émission de lumière de fluorescence ou de luminescence d'une particule mobile dans un échantillon, la particule émettant la lumière de fluorescence ou de luminescence dans une bande spectrale d'émission , le procédé comportant :

- a) illumination de l'échantillon dans une bande spectrale de détection, et formation d'une image de détection de l'échantillon, dans la bande spectrale de détection, la bande spectrale de détection étant différente de la bande spectrale d'émission ;
- b) formation d'une image d'émission, de l'échantillon, dans la bande spectrale d'émission;
- l'image de détection et l'image d'émission étant obtenues à partir d'une acquisition d'une image de l'échantillon par un capteur d'image, dans la bande spectrale de détection et dans la bande spectrale d'émission,
- les étapes a) et b) étant réitérées ;
- le procédé comportant également les étapes suivantes :

  - c) à partir de chaque image de détection ($I_d$) résultant de chaque itération des étapes a) et b), détection successivement acquise de la particule et détermination d'une région d'intérêt autour de la particule ;
  - d) à partir de la région d'intérêt résultant de chaque étape c), extraction d'une région d'intérêt dans chaque image d'émission de l'échantillon successivement acquise, la région d'intérêt extraite dans chaque image d'émission correspondant à la même particule détectée dans chaque image de détection successivement acquise ;
  - e) sommation des régions d'intérêt extraites dans chaque étape d), de façon à former une image d'émission intégrée de la particule, représentative de la fluorescence ou de la luminescence de la particule, l'image d'émission intégrée de la particule étant soit une image obtenue par l'intégration de plusieurs régions d'intérêt extraites de l'étape d), soit un nombre correspondant à un cumul des intensités des images de fluorescence successives, dans les régions extraites de l'étape d).

Selon un mode de réalisation :

- la particule émet une lumière de fluorescence dans la bande spectrale d'émission lorsqu'elle est illuminée dans une bande spectrale d'excitation :
- l'étape b) comporte une illumination de l'échantillon dans la bande spectrale d'excitation ;
- la bande spectrale de détection est distante de la bande spectrale d'excitation
- le capteur d'image est couplé à un filtre, de façon à bloquer la bande spectrale d'excitation ;

Les étapes a) et b) peuvent être mises en œuvre simultanément.

Les étapes c), d) et e) peuvent être mises en œuvre au cours de chaque itération des étapes a) et b) ou à la suite des itérations des étapes a) et b).

Au cours de chaque itération, les étapes a) et b) peuvent être mises en œuvre successivement Selon une possibilité:

- au moins deux itérations des étapes a) et b) sont effectuées successivement, selon une itération de rang n et une itération de rang n+1, n étant un entier strictement positif ;
- l'étape d), comporte :

  (i) une interpolation des positions de la particule dans les images de détection résultant des étapes a) des itérations de rang n et n+1, de façon à estimer une position interpolée de la particule lors de l'étape b) de l'itération de rang n ;
  (ii) une extraction de la région d'intérêt dans l'image d'émission formée lors de l'itération de rang n à partir de la position interpolée estimée lors de la sous-étape (ii).

Selon un mode de réalisation :

- le capteur d'image est un capteur d'image couleur ;
- dans l'étape a), l'image de détection de l'échantillon est obtenue à partir d'une première composante spectrale d'une l'image acquise par le capteur d'image, la première composante spectrale correspondant à tout ou partie de la bande spectrale de détection ;
- et/ou dans l'étape b), l'image d'émission de l'échantillon est obtenue à partir d'une deuxième composante spectrale de l'image acquise par le capteur d'image, la deuxième composante spectrale correspondant à tout ou partie de la bande spectrale d'émission ;
- de façon que la même image, acquise par le capteur d'image, permet de former l'image de détection et l'image d'émission de l'échantillon.

Selon un mode de réalisation :

- le capteur d'image comporte un premier capteur d'image élémentaire et un deuxième capteur d'image élémentaire, ainsi qu'un séparateur, le séparateur étant configuré pour envoyer la lumière,

  - dans la bande spectrale de détection, vers le premier capteur d'image élémentaire ;
  - dans la bande spectrale d'émission, vers le deuxième capteur d'image élémentaire ;

- lors de chaque étape a), l'image de détection, dans bande spectrale de détection, est acquise par le premier capteur d'image élémentaire ;
- lors de chaque étape b), l'image d'émission, dans la bande spectrale d'émission, est acquise par le deuxième capteur d'image élémentaire.

Selon une possibilité, l'étape e) comporte le calcul d'une moyenne des régions d'intérêt extraites dans chaque image d'émission de l'échantillon, correspondant à la même particule.

Selon une possibilité, l'étape e) est mise en œuvre au cours de chaque itération des étapes a) et b).

Selon une possibilité :

- le critère d'arrêt est un nombre d'itérations prédéterminé ;
- ou l'étape e) étant mise en œuvre au cours de chaque itération des étapes a) et b), le critère d'arrêt est une obtention d'une image d'émission intégrée dans laquelle le rapport signal sur bruit dépasse un seuil prédéterminé.
- ou l'étape e) étant mise en œuvre au cours de chaque itération des étapes a) et b), le procédé comporte un affichage de l'image d'émission intégrée , les itérations étant arrêtées par un utilisateur.

Selon une possibilité, lors de chaque étape a), l'intensité de l'illumination de l'échantillon, dans la bande spectrale de détection, est ajustée de façon que le rapport signal à bruit de l'image de détection soit inférieur à une valeur prédéterminée.

Un deuxième objet de l'invention est un dispositif d'observation d'un échantillon comportant une particule mobile, la particule étant susceptible d'émettre une lumière de fluorescence ou de luminescence dans une bande spectrale d'émission, le dispositif comportant :

- une source de lumière de détection, configurée pour illuminer l'échantillon dans une bande spectrale de détection, distante de la bande spectrale d'émission;
- un capteur d'image configuré pour acquérir une image de l'échantillon, dans la bande spectrale de détection et la bande spectrale d'émission ;
- le dispositif étant configuré pour maintenir l'échantillon face au capteur d'image, selon un plan d'échantillon ;
- un filtre d'émission, disposé entre le capteur d'image et le plan d'échantillon, le filtre d'émission étant configuré pour transmettre la lumière dans la bande spectrale de détection et dans la bande spectrale d'émission ;
- une unité de traitement (30), configurée pour former, à partir de l'image acquise par le capteur d'image ;

  - une image de détection de l'échantillon, dans la bande spectrale de détection ;
  - une image d'émission de l'échantillon, dans la bande spectrale d'émission ;

- le dispositif étant tel que l'unité de traitement est programmée pour mettre en œuvre les étapes a) à e) d'un procédé selon le premier objet de l'invention.

Le dispositif peut comporter une source de lumière d'excitation, configurée pour illuminer l'échantillon dans une bande spectrale d'excitation, distante de la bande spectrale d'émission et de la bande spectrale de détection.

Selon une possibilité, la source de lumière de détection et la source de lumière d'excitation sont configurées pour être activées simultanément ou séquentiellement, selon un décalage temporel inférieur à 100 ms ou à 10 ms.

Selon une possibilité, le capteur d'image est un capteur d'image couleur.

Selon une possibilité, le capteur d'image comporte un premier capteur d'image élémentaire et un deuxième capteur d'image élémentaire, ainsi qu'un séparateur, le séparateur étant configuré pour envoyer la lumière,

- dans la bande spectrale de détection, vers le premier capteur d'image élémentaire;
- dans la bande spectrale d'émission, vers le deuxième capteur d'image élémentaire.

L'invention sera mieux comprise à la lecture de l'exposé des exemples de réalisation présentés, dans la suite de la description, en lien avec les figures listées ci-dessous.

**FIGURES**

La figure 1 représente un échantillon comportant des spermatozoïdes.

La figure 2A montre un premier mode de réalisation d'un dispositif selon l'invention.

La figure 2B montre un deuxième mode de réalisation d'un dispositif selon l'invention.

La figure 3 montre des bandes passantes de transmission de différents filtres pouvant être utilisés dans le dispositif.

La figure 4A montre une image de spermatozoïdes dans une bande spectrale de détection.

La figure 4B montre une image de spermatozoïdes dans une bande spectrale de fluorescence.

La figure 5A montre un enchainement d'étapes de traitement d'images de détection et de fluorescence d'un échantillon.

La figure 5B montre une autre possibilité d'enchainement d'étapes de traitement d'images de détection et de fluorescence d'un échantillon.

La figure 5C montre une autre possibilité d'enchainement d'étapes de traitement d'images de détection et de fluorescence d'un échantillon.

La figure 6A montre une région d'intérêt correspondant à un spermatozoïde, déterminée à partir d'une image de détection.

La figure 6B montre la région d'intérêt, définie sur l'image 6A, appliquée à une image de fluorescence.

La figure 7 montre différentes régions d'intérêt formées sur différentes images de détection (colonne de gauche) et extraites de différentes images de fluorescence (colonne de droite). Chaque région d'intérêt correspond à un même spermatozoïde. Chaque ligne de cette figure montre une image de détection et une image de fluorescence simultanément obtenues.

Les figures 8A, 8B, 8C et 8D illustrent la formation d'une image de fluorescence intégrée d'un spermatozoïde.

La figure 9A schématise un mode de réalisation, dans lequel les images de détection et de fluorescence résultent d'images acquises successivement par le capteur d'image.

La figure 9B illustre une estimation de la position d'une région d'intérêt, correspondant à un spermatozoïde, à un instant, à partir des positions du spermatozoïde déterminées à partir d'images de détection acquises antérieurement et postérieurement audit instant. Sur la figure 9B, les positions encerclées correspondent à des positions vraies. Les positions représentées par une croix sont des positions interpolées à partir de deux positions vraies respectivement précédente et suivante.

Les figures 10A, 10B, 10C, 10D et 10E montrent un exemple d'application de l'invention à la détermination de l'état (vivant ou mort) d'un spermatozoïde.

Les figures 11A, 11B, 11C, 11D et 11E montrent un autre exemple d'application de l'invention à la détermination de l'état (vivant ou mort) d'un spermatozoïde.

**EXPOSE DE MODES DE REALISATION PARTICULIERS**

La figure 2A représente un exemple de dispositif 1 permettant de mettre en œuvre l'invention. Le dispositif est destiné à observer un échantillon 2, comportant des particules mobiles. Dans cet exemple, de façon non limitative, les particules sont des particules biologiques, en particulier des spermatozoïdes. Les spermatozoïdes sont marqués avec un fluorophore. Le fluorophore peut être un marqueur de viabilité, ou un marqueur de sexage, un marqueur d'apoptose, ou un marqueur physiologique. Le fluorophore émet une lumière de fluorescence, dans une bande spectrale d'émission de fluorescence $\Delta\lambda m$, lorsqu'il est illuminé dans une bande spectrale d'excitation $\Delta\lambda e$. Les spermatozoïdes peuvent être marqués avec différents fluorophores, chaque fluorophore présentant une bande spectrale d'excitation $\Delta\lambda e$ et une bande spectrale d'émission de fluorescence $\Delta\lambda m$. Pour un même fluorophore, la bande spectrale d'émission de fluorescence $\Delta\lambda m$ est distante de la bande spectrale d'excitation $\Delta\lambda e$, le recouvrement entre la bande spectrale d'émission de fluorescence $\Delta\lambda m$ et la bande spectrale d'excitation étant considéré comme nul.

Le dispositif comporte :

- une source de lumière de détection 11, configurée pour émettre une lumière dans une bande spectrale de détection Δλd. La bande spectrale de détection Δλd est différente et distante de la bande spectrale d'excitation Δλe et de la bande spectrale d'émission de fluorescence Δλm du fluorophore marquant le spermatozoïde ;
- une source de lumière d'excitation 12, configurée pour émettre une lumière d'excitation dans la bande spectrale d'excitation Δλe du fluorophore ;
- un capteur d'image 20, destiné à acquérir une image de l'échantillon. Le capteur d'image 20 est un capteur d'image couleur, permettant de former une image selon différentes composantes spectrales. Le capteur d'image comporte des pixels, formant une matrice de pixels, s'étendant selon un plan de détection.
- un objectif 16, configuré pour concentrer la lumière d'excitation, réfléchie par le cube séparateur 15, sur l'échantillon. L'objectif 16 définit un plan image et un plan objet. De préférence, le plan objet s'étend dans l'échantillon et le plan image correspond au plan de détection du capteur d'image 20. Selon une possibilité, dite d'imagerie défocalisée, le plan de détection est légèrement décalé par rapport au plan image de l'objectif 16, et/ou le plan objet est légèrement décalé par rapport à l'échantillon. Une telle modalité permet de former une image holographique. Moyennant le recours à des algorithmes de reconstruction appropriés, on peut alors obtenir une image de phase de l'échantillon. L'imagerie défocalisée est généralement utilisée lorsque les particules de l'échantillon sont transparentes ou considérées comme telles. Cependant, pour l'observation de la lumière de fluorescence, il est préférable d'opter pour la configuration focalisée, selon laquelle l'échantillon s'étend dans le plan objet de l'objectif et le capteur d'image s'étend selon le plan image de l'objectif. La configuration défocalisée peut être mise en œuvre pour former les images de détection décrites par la suite.

Dans l'exemple représenté, le dispositif comporte :

- un cube séparateur 15, comportant une lame dichroïque, permettant de renvoyer la lumière d'excitation vers l'échantillon 2, à travers l'objectif 16 ;
- une lentille convergente 13, disposée face à la deuxième source de lumière 12. La lentille convergente permet de mettre en forme la lumière d'excitation vers le cube séparateur 15 ;
- une lentille convergente 19, permettant de focaliser la lumière issue de l'échantillon vers le capteur d'image.

Le dispositif peut comporter des filtres, permettant d'ajuster la bande spectrale de détection Δλd, la bande spectrale d'excitation Δλe, ainsi que pour définir les bandes spectrales qui atteignent le capteur d'image 20. Le dispositif est configuré de façon que le capteur d'image ne puisse pas détecter la bande spectrale d'excitation.

Dans cet exemple, un filtre de détection 11' est disposé entre la source de lumière de détection 11 et l'échantillon. Le filtre 11 de détection est configuré pour définir la bande spectrale de détection Δλd. Le recours au filtre de détection est optionnel, en particulier lorsque la source de lumière 11 émet la lumière selon une bande spectrale de détection bien définie, ce qui est par exemple le cas d'une diode électroluminescente (LED).

Le dispositif comporte un filtre d'excitation 14, destiné à délimiter la bande spectrale d'excitation Δλe de la lumière émise par la source de lumière d'excitation 12.

Le dispositif comporte un filtre d'émission 18, destiné à bloquer la (ou chaque) bande spectrale d'excitation, de façon à rendre le capteur d'image insensible à cette dernière. Le filtre d'émission 18 est passant pour la bande spectrale de détection Δλd ainsi que pour la (ou chaque) bande spectrale d'émission de fluorescence Δλm. Le filtre d'émission est disposé entre le cube séparateur 15 et la lentille 19.

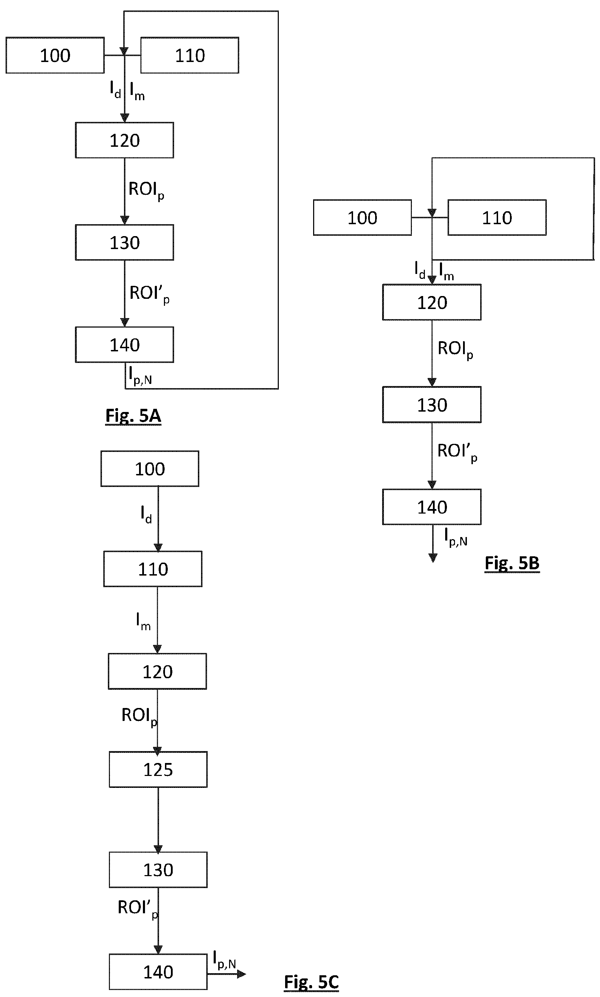

Le dispositif comporte une unité de traitement 30, comportant un microprocesseur, programmée pour effectuer des opérations de traitement d'image décrites par la suite, en lien avec les figures 5A à 5C. Les opérations de traitement d'image sont programmées et stockées dans une mémoire 31, à laquelle l'unité de traitement est reliée. L'unité de traitement peut être connectée à un écran 32.

Dans le dispositif décrit en lien avec la figure 2A, la source de lumière de détection 11 est distincte de la source de lumière d'excitation 12. L'intensité d'émission de la source de lumière de détection 11 peut être ajustée pour éviter qu'une partie de l'intensité de la source de lumière de détection soit détectée dans la bande spectrale d'émission Δλm, par diaphonie (cross-talk). L'ajustement consiste à obtenir un rapport signal sur bruit suffisant pour détecter correctement la position de chaque spermatozoïde sur les images de détection. Il s'agit de « minimiser » l'intensité de source de lumière de détection de façon à obtenir un rapport signal sur bruit dépassant un seuil acceptable.

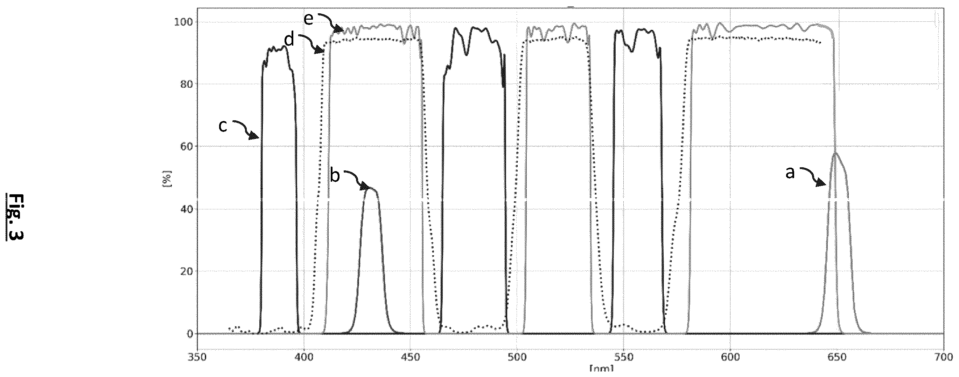

La figure 3 représente différentes bandes passantes de filtres pouvant être utilisés. La courbe a représente la bande spectrale d'un filtre de détection 11' (référence Thorlabs 650-10) définissant une bande passante centrée sur 650 nm. La courbe b représente la bande spectrale d'un autre filtre de détection 11' (référence Thorlabs 430-10) définissant une bande passante centrée sur 430 nm. La courbe c montre la bande spectrale d'un filtre d'excitation 14. La courbe d montre la bande spectrale de transmission du miroir dichroïque inclus dans le cube 15. La courbe e montre la bande spectrale de transmission du filtre d'émission 18.

Les courbes c, d et e sont obtenues avec un jeu de filtres Semrock DA/FI/TR/3X-A. Les courbes c, d e illustrent la possibilité de définir simultanément plusieurs bandes spectrales d'excitation $\Delta\lambda e$ (cf. bandes passantes de la courbe c) et plusieurs bandes spectrales d'émission de fluorescence $\Delta\lambda m$ (cf. bandes passantes des courbes d et e, qui bloquent les bandes spectrales d'excitation). Cela permet d'adresser simultanément plusieurs fluorophores, présentant des bandes spectrales de fluorescence $\Delta\lambda m$ différentes les unes des autres. Cela permet également d'utiliser une des bandes passantes, définies par le filtre d'émission, pour transmettre bande spectrale de détection $\Delta\lambda d$.

Dans le mode de réalisation représenté sur la figure 2A, le capteur d'image 20 est un capteur d'image couleur. Ainsi, c'est le même capteur d'image qui permet l'acquisition d'une l'image de détection $I_d$, dans la bande spectrale de détection $\Delta\lambda d$ et d'une image d'émission de fluorescence $I_m$ dans la bande spectrale d'émission de fluorescence $\Delta\lambda m$.

Par image de détection $I_d$, on entend une image permettant de détecter la position du spermatozoïde dans l'échantillon. L'image de détection $I_d$ correspond à une composante spectrale de l'image acquise par le capteur d'image, dans la bande spectrale de détection $\Delta\lambda d$.

L'image d'émission de fluorescence $I_m$ correspond à une composante spectrale de l'image acquise par le capteur d'image, dans la bande spectrale d'émission de fluorescence $\Delta\lambda m$. Lorsque l'on souhaite adresser simultanément plusieurs images d'émission de fluorescence $I_m$, chaque image d'émission fluorescence correspond à une composante spectrale de l'image acquise par le capteur d'image, dans une bande spectrale d'émission de fluorescence $\Delta\lambda m$.

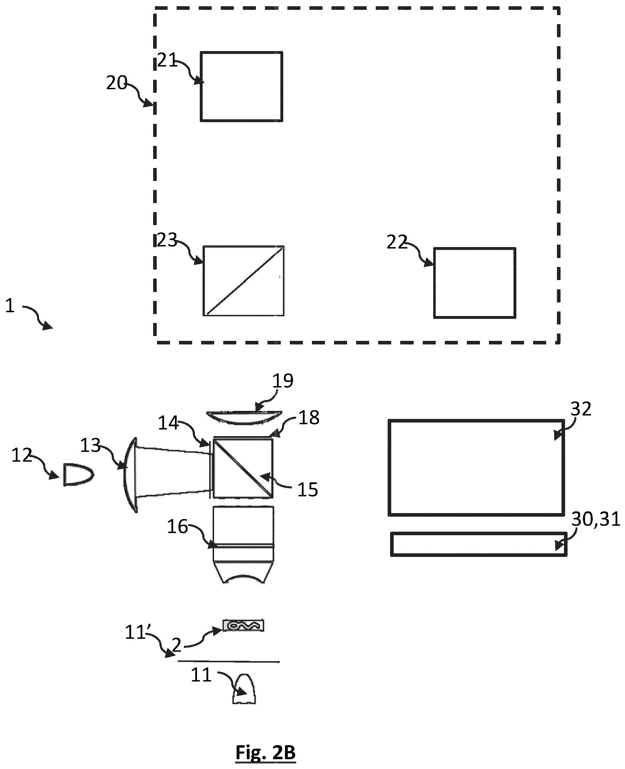

La figure 2B représente un autre exemple de dispositif selon l'invention, dans lequel le capteur d'image 20 est formé d'un premier capteur d'image élémentaire 21 et un deuxième capteur d'image élémentaire 22, séparés l'un de l'autre. Selon cette configuration, le capteur d'image comporte un séparateur 23 configuré pour :

- diriger la lumière émise par l'échantillon, dans la bande spectrale de détection $\Delta\lambda d$, vers le premier capteur d'image élémentaire 21 ;
- diriger la lumière émise par l'échantillon, dans la bande spectrale d'émission de fluorescence $\Delta\lambda m$, vers le deuxième capteur d'image élémentaire 22.

L'image de détection $I_d$ correspond à l'image acquise par le premier capteur d'image élémentaire 21. L'image d'émission de fluorescence $I_m$ correspond à l'image acquise par le deuxième capteur d'image élémentaire 22.

L'inventeur a mis en œuvre une configuration telle que décrite en lien avec la figure 2A, le capteur d'image couleur étant un capteur RGB IDS UI-3160CP-C-HQ. L'objectif 16 était un objectif de grandissement x20 - ouverture numérique 0.4 - référence Olympus Plan Achromat 20x/0.4NA. On a utilisé le jeu de filtres décrit en lien avec la figure 3. L'échantillon 2 comportait des spermatozoïdes.

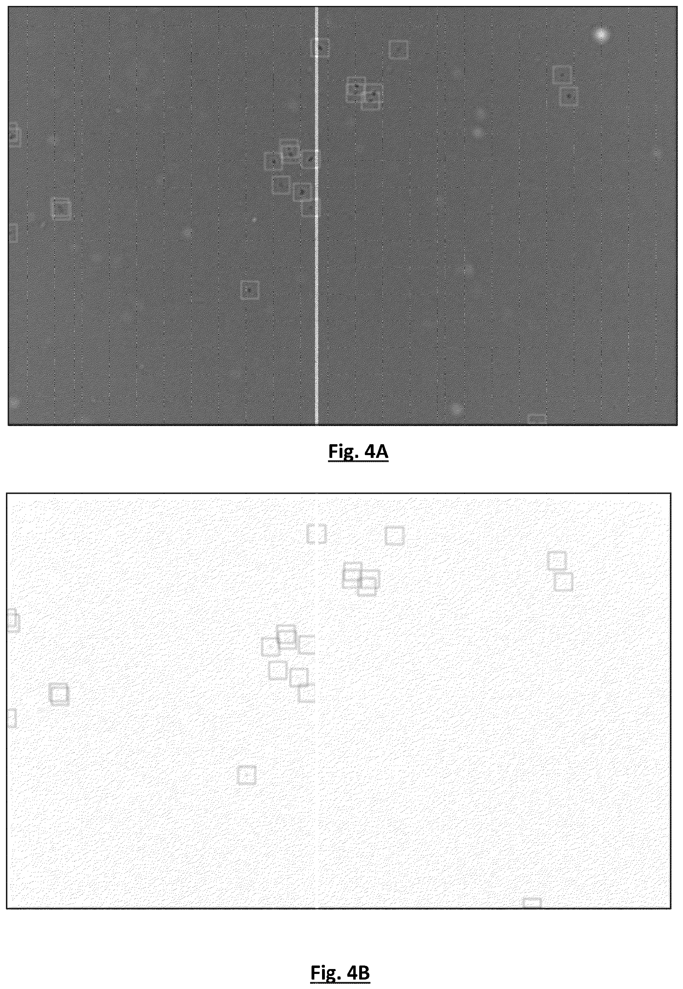

Dans les figures 4A, 4B, 6A, 6B, 7, 8A, 8B, 8C, on a représenté les images inversées : les niveaux de gris sombres correspondent aux forts niveaux d'intensité lumineuse.

Dans une première série d'essais, la bande spectrale de détection $\Delta\lambda d$ était dans la bande spectrale rouge, centrée sur 650 nm (Filtre Thorlabs FB650-10). Les spermatozoïdes étaient marqués avec un marqueur fluorescent Hoechst. La source de lumière d'excitation 12 était une LED émettant à 375 nm. Afin de tenir compte du mouvement des spermatozoïdes, les images ont été acquises selon un temps de pose faible : 10 ms.

La figure 4A montre un exemple d'une image de détection $I_d$ formée, à partir de l'image acquise par le capteur d'image, dans une composante spectrale correspondant à la bande spectrale de détection $\Delta\lambda d$ centrée sur 650 nm. La figure 4B montre l'image de fluorescence $I_m$ formée, à partir de l'image acquise par le capteur d'image, dans une composante spectrale correspondant la bande spectrale d'émission de fluorescence $\Delta\lambda m$.

L'image de détection $I_d$ et l'image de fluorescence $I_m$ correspondent aux composantes d'une même image acquise par le capteur d'image, respectivement dans la bande spectrale de détection $\Delta\lambda d$ et la bande spectrale d'émission de fluorescence $\Delta\lambda m$.

Sur l'image de détection (cf. figure 4A), on a détecté la position de chaque spermatozoïde. Autour de chaque spermatozoïde, une région d'intérêt ROI a été définie, qui est matérialisée sur la figure 4A par des cadres clairs.

On observe que le signal de fluorescence est particulièrement faible : cf. figure 4B. Une seule image d'émission de fluorescence n'est pas suffisante pour former une image de qualité acceptable. C'est la conséquence du faible temps de pose, ainsi que du faible rendement de fluorescence. Sur la figure 4B, on a représenté les régions d'intérêt ROI' extraites, sur l'image d'émission de fluorescence, à partir des positions des spermatozoïdes obtenues sur l'image de détection (fig. 4A).

Afin de disposer d'un signal de fluorescence plus exploitable de chaque spermatozoïde, il est nécessaire d'intégrer le signal de fluorescence émis par chaque spermatozoïde selon une durée plus longue. Il est donc nécessaire de surmonter la difficulté liée aux mouvements des spermatozoïdes. C'est l'objet des étapes 100 à 140 schématisées sur les figures 5A à 5C.

Etape 100 : illumination de l'échantillon dans la bande spectrale de détection, à l'aide de la source de lumière de détection 11 et acquisition, par le capteur d'image, d'une image de détection de l'échantillon dans la bande spectrale de détection $\Delta\lambda d$. Dans cet exemple, le capteur d'image est un capteur d'image couleur, le dispositif utilisé étant celui décrit en lien avec la figure 2A.

Etape 110 : illumination de l'échantillon dans la bande spectrale d'excitation $\Delta\lambda e$, à l'aide de la source de lumière d'excitation 12, et acquisition, par le capteur d'image, d'une image de fluorescence de l'échantillon, dans la bande spectrale d'émission de fluorescence $\Delta\lambda m$. Il s'agit du même capteur d'image que celui utilisé lors de l'étape 100.

Dans cet exemple, les étapes 100 et 110 sont effectuées simultanément. L'échantillon est illuminé de façon continue par la source de lumière de détection et par la source de lumière d'excitation. Une même image I est acquise, simultanément dans la bande spectrale de détection $\Delta\lambda d$ et dans la bande spectrale d'émission de fluorescence $\Delta\lambda m$. Il s'agit d'un mode de réalisation avantageux, car il ne nécessite qu'un seul capteur d'image couleur.

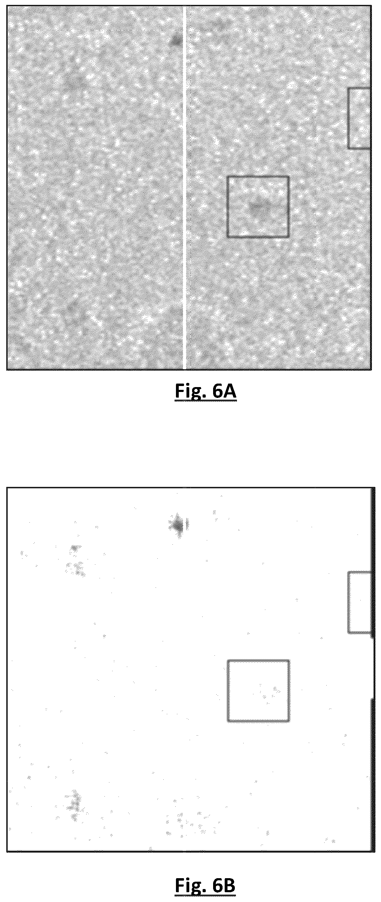

Etape 120 : formation d'une image de détection $I_d$ de l'échantillon, à partir de l'image I acquise par le capteur d'image. L'image de détection $I_d$ correspond à une première composante spectrale, dans la bande spectrale de détection $\Delta\lambda d$, de l'image I acquise par le capteur d'image. A partir de l'image de détection $I_d$, détection des spermatozoïdes et détermination d'une région d'intérêt (ROI) autour de chaque spermatozoïde. Une telle étape peut être réalisée avec un algorithme classique de suivi de particules. Dans cet exemple, chaque région d'intérêt ROI est un carré de 50 pixels de côté. La figure 6A représente un détail d'une image de détection $I_d$, sur laquelle on a matérialisé une région d'intérêt $ROI_p$ centrée sur un spermatozoïde. On a mis en œuvre un algorithme de suivi (tracking) des particules, ce qui permet de suivre chaque spermatozoïde individuellement entre différentes images de détection. Chaque région d'intérêt ROI est annotée d'un identifiant p désignant le spermatozoïde. Par la suite, chaque région d'intérêt est notée $ROI_p$, où p correspond à l'annotation de la région d'intérêt.

Etape 130 : formation d'une image d'émission de fluorescence $I_m$ de l'échantillon, à partir de l'image I acquise par le capteur d'image. L'image d'émission fluorescence $I_m$ correspond à une deuxième composante spectrale, dans la bande spectrale de fluorescence $\Delta\lambda m$, de l'image I acquise par le capteur d'image. A partir des régions d'intérêt annotées $ROI_p$ de l'image de détection résultant de l'étape 120, extraction d'une région d'intérêt dite jumelle $ROI'_p$ sur l'image de fluorescence. Par région d'intérêt jumelle, on entend une région d'intérêt, extraite dans l'image de fluorescence, qui correspond à la région d'intérêt déterminée, dans l'étape 120, dans l'image de détection. La région d'intérêt $ROI_p$ formée dans l'image de détection $I_d$ et la région d'intérêt jumelle $ROI'_p$ extraite dans l'image de fluorescence $I_m$ correspondent à la même particule p.

Les images de détection et de fluorescence sont issues d'une même image I acquise par le capteur d'image 20. Chaque région d'intérêt $ROI_p$ définie sur l'image de détection, lors de l'étape 120, est utilisée pour extraire une région d'intérêt jumelle $ROI'_p$ dans l'image de fluorescence. La figure 6B montre une région d'intérêt jumelle $ROI'_p$ de la région d'intérêt $ROI_p$ définie sur la figure 6A.

L'avantage d'utiliser un même capteur d'image est de former, à partir d'une même image acquise I, l'image de détection $I_d$ et l'image de fluorescence $I_m$. Les régions d'intérêt $ROI_p$, $ROI'_p$, correspondant à une même particule p, sont identiques sur chaque couple d'images (image de détection $I_d$, image de fluorescence $I_m$).

Lorsqu'on utilise un capteur d'image comportant deux capteurs d'images élémentaires, comme décrit en lien avec la figure 2B, les capteurs d'image élémentaires sont de préférence identiques et synchronisés, de façon à faciliter la correspondance entre une région d'intérêt $ROI_p$ définie sur l'image de détection $I_d$, pour un spermatozoïde, et la région d'intérêt jumelle $ROI'_p$ extraite de l'image de fluorescence $I_m$ pour le même spermatozoïde.

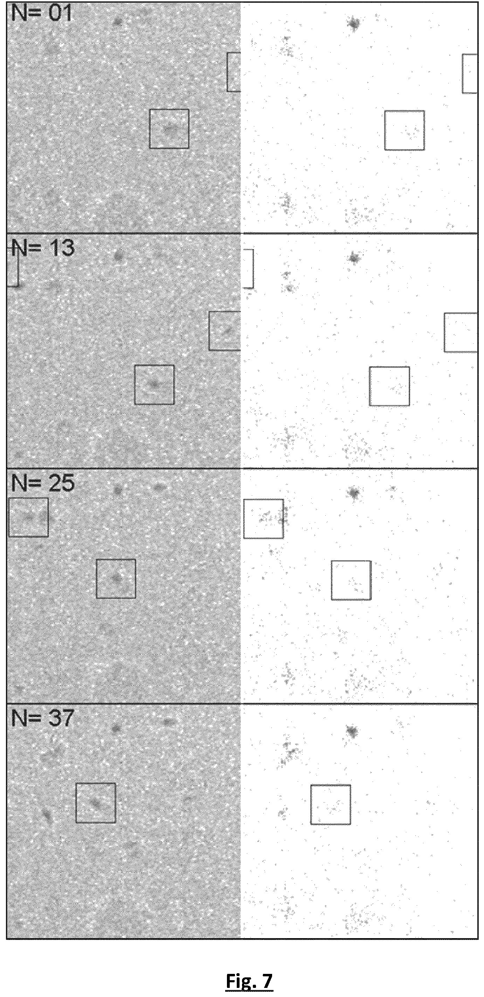

Sur la figure 7, on a représenté différentes images de détection $I_d$ (colonne de gauche) et différentes images de fluorescence $I_m$ (colonne de droite). Sur chaque image de détection $I_d$, on a suivi la trajectoire de spermatozoïdes, et défini une région d'intérêt autour de chaque spermatozoïde détecté. Les régions d'intérêt définies à partir de chaque image de détection $I_d$ ont été appliquées pour extraire des régions d'intérêt jumelles sur chaque image de fluorescence $I_m$ : cf. colonne de droite. Comme précédemment décrit, les régions d'intérêt définies sur chaque image sont annotées de façon que sur les images de détection et sur les images de fluorescence, chaque annotation correspond à un même spermatozoïde.

A chaque itération des étapes 100 à 130 peut être assigné un rang n. n est un entier naturel strictement positif. La figure 7 représente l'image de détection et l'image de fluorescence formées à partir d'images acquises lors des itérations de rangs 1, 13, 25 et 37.

Etape 140 : au cours de cette étape, on somme les régions d'intérêt jumelles $ROI'_p$, ayant la même annotation, de chaque image de fluorescence $I_m$ résultant de l'étape 130. Cela permet de former une image de fluorescence intégrée de chaque spermatozoïde. Par image intégrée, on entend une image obtenue par intégration de plusieurs images.

Ainsi,

$$I_{p,N} = \sum_1^N ROI'_{p,n} \quad (1)$$

où :

- $ROI'_{p,n}$ est une région d'intérêt jumelle correspondant au spermatozoïde p défini sur l'image de fluorescence acquise au cours de l'itération n ;

- *N* correspond au nombre total d'itérations ;
- $I_{p,N}$ est l'image de fluorescence intégrée après *N* itérations.

Pour chaque spermatozoïde, plus le nombre d'images de fluorescence cumulées augmente, plus le rapport signal sur bruit augmente également.

D'une façon générale, l'étape 140 revient à former un signal de fluorescence intégré, à partir de régions d'intérêt extraites d'images de fluorescence successives, et correspondant à une même particule. Le signal de fluorescence peut être l'image de fluorescence intégrée $I_{p,N}$, correspondant au cumul des régions d'intérêt extraites sur plusieurs images de fluorescence successives. Il peut également s'agir d'un nombre correspondant à un cumul des intensités des images de fluorescence successives $I_m$, dans les régions d'intérêt extraites de chacune d'entre elle, et correspondant à la même particule.

Selon une possibilité, on effectue une moyenne des régions d'intérêt, de même annotation, extraites d'images de fluorescence formées successivement. Le fait d'effectuer une moyenne permet de conserver une même dynamique pour chaque image de fluorescence.

Ainsi,

$$I_{p,N} = \frac{\sum_1^N ROI'_{p,n}}{N} \quad (2)$$

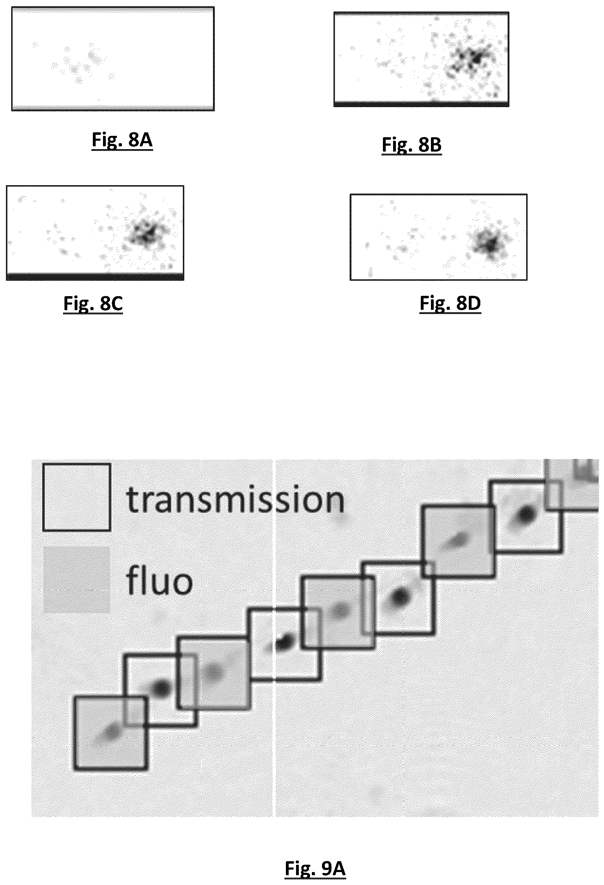

Les figures 8A, 8B, 8C et 8D représentent respectivement, pour un même spermatozoïde p, les images de fluorescence intégrées $I_{p,N}$, telles que définies selon (2), correspondant, respectivement obtenues après N = 1 itération (figure 8A), N=13 itérations (figure 8B), N=25 itérations (figure 8C) et N=47 itérations (figure 8D). On peut visuellement observer l'amélioration du rapport signal sur bruit en fonction du nombre d'itérations, c'est-à-dire du nombre d'images de fluorescence moyennées.

Les étapes 100 à 140 sont réitérées, les images de l'échantillon étant acquises selon une fréquence d'acquisition élevée, par exemple à 60 images par seconde. Sur la figure 5A, on a considéré que le l'image de fluorescence intégrée $I_{p,N}$, pour chaque spermatozoïde p, est renouvelée au cours de chaque itération. Chaque itération comporte les étapes 100 à 140. Les itérations se poursuivent jusqu'à l'atteinte d'un critère d'arrêt : il peut s'agir d'un nombre d'itérations prédéterminé. Le critère d'arrêt peut être défini en fonction du rapport signal sur bruit obtenu sur l'image de fluorescence intégrée associée à chaque spermatozoïde : au-delà d'un seuil de rapport signal sur bruit prédéfini, les itérations cessent pour le spermatozoïde dont l'image de fluorescence intégrée qui lui est associée dépasse le seuil de rapport signal sur bruit. L'arrêt des itérations peut également être décidé par l'utilisateur, en fonction du rendu visuel de chaque image de fluorescence intégrée.

Selon une autre possibilité, les étapes 100 et 110 sont réitérées. Les étapes 120 à 140 sont effectuées en post-traitement, après une itération d'un nombre prédéterminé d'itérations des étapes 100 à 110. Cf. figure 5B.

Il est préférable que les images de détection et les images de fluorescence soient formées à partir d'une même image, ou soient acquises simultanément si l'on utilise plusieurs capteurs élémentaires. Cependant, il peut être envisagé que les acquisitions respectives d'images, permettant la formation d'images de détection et d'images de fluorescence, soient alternées. Il est préférable de limiter l'intervalle de temps entre les acquisitions successives d'une image de détection et d'une image de fluorescence, compte tenu du déplacement des spermatozoïdes. La durée entre deux acquisitions successives peut être telle que le déplacement des spermatozoïdes, entre une image de fluorescence et une image de détection, soit considéré comme négligeable. Cela facilite la correspondance entre les régions d'intérêt $ROI_p$ déterminées sur les images de détection et les régions d'intérêt déterminées sur les images de fluorescence. De façon alternative, la position des spermatozoïdes entre deux images de détection successives, peut être estimée par interpolation, comme décrit par la suite

Le fait d'alterner des images de détection et des images de fluorescence permet d'utiliser un capteur d'image monochrome, ce qui est considéré comme plus usuel dans les applications de microscopie.

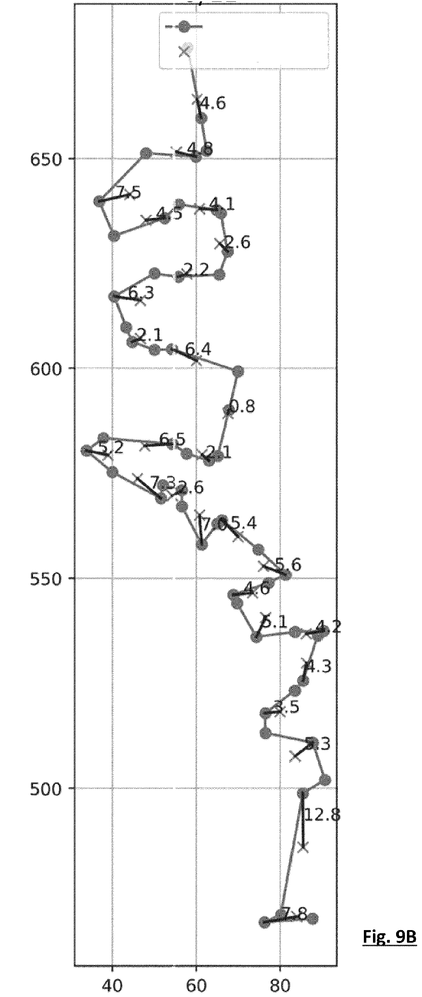

Selon une possibilité, illustrée sur les figures 5C, 9A et 9B, les acquisitions des images de détection et des images de fluorescence sont alternées. Ainsi, au cours d'une même itération, les étapes 100 et 110 sont temporellement décalées l'une par rapport à l'autre. Dans ce cas, le procédé peut comporter une étape d'interpolation 125. Les régions d'intérêt $ROI'_{p,n}$ extraites d'une image de fluorescence, dans une itération de rang n, sont obtenues par interpolation de régions d'intérêt $ROI_{p,n}$ et $ROI_{p,n+1}$ correspondant respectivement au spermatozoïde p et déterminées sur les images de détection de rang n et n+1.

Sur la figure 9A, on a représenté les régions d'intérêt $ROI_{p,n}$ et $ROI_{p,n+1}$ détectées sur des images de détection successives de rang n et n+1, et ainsi que la région d'intérêt interpolée $ROI'_{p,n}$, assignée à l'image de fluorescence acquise entre lesdites images de détection.

La figure 9B représente une évolution du point central de régions d'intérêt $ROI_{p,n}$ détectées sur différentes images de détection successives. Chaque point, matérialisé par un cercle, correspond à une position du point central. Afin de tester l'algorithme d'interpolation, on a estimé la position de points centraux de régions d'intérêt $ROI_{p,n+1}$ entre deux régions d'intérêt $ROI_{p,n}$, $ROI_{p,n+2}$. Les positions interpolées des points centraux des régions d'intérêt $ROI_{p,n+1}$ sont matérialisées par des croix X. L'écart entre la position réelle (cercle) et la position interpolée (croix) est matérialisé par un segment linéaire. On observe que plus la trajectoire du spermatozoïde est droite, plus l'interpolation est correcte.

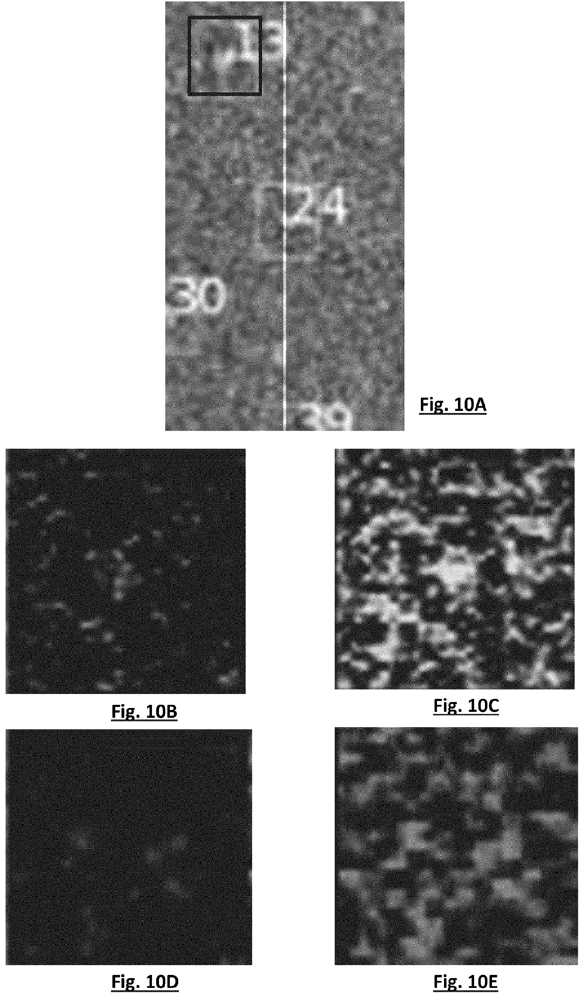

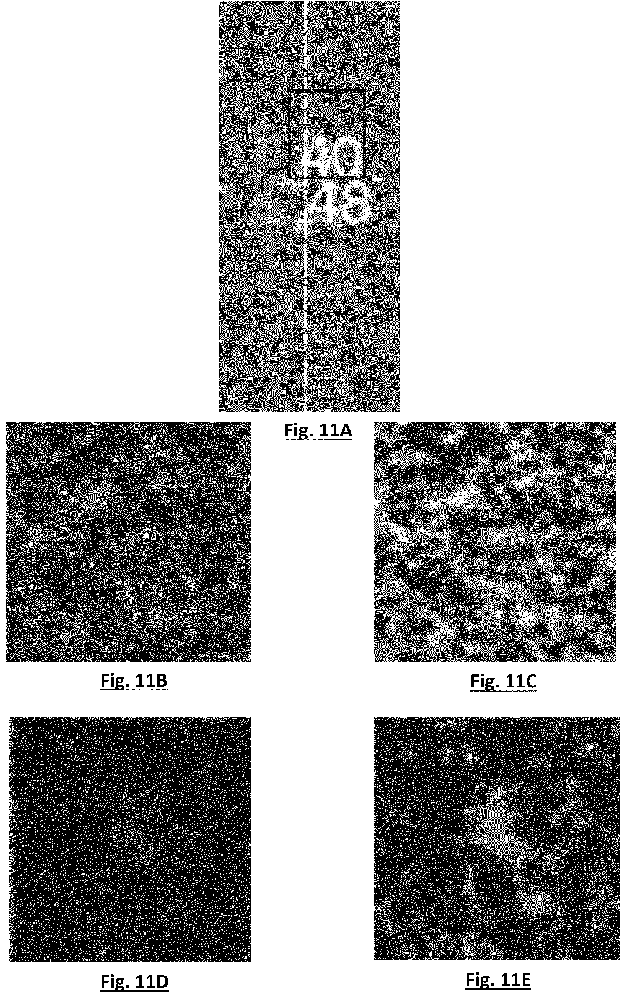

Les figures 10A à 10E et 11A à 11E illustrent la possibilité d'appliquer l'invention à deux mesures simultanées de fluorescence. Pour obtenir ces images, on a utilisé un dispositif tel que schématisé sur la figure 2A. Les spermatozoïdes ont été marqués avec un kit de fluorophores (easy-kit viability - fournisseur IMV) permettant de déterminer si le spermatozoïde est mort ou vivant. Les fluorophores utilisés étaient SYBR green (Thermo fisher) et l'iodure de Propidium (Propidium Iodide PI). Ainsi, Les spermatozoïdes vivants émettent une lumière de fluorescence verte et les spermatozoïdes morts émettent une lumière de fluorescence rouge. La bande spectrale d'excitation était centrée sur 450 nm. La bande spectrale de détection $\Delta\lambda d$ était centrée sur la longueur d'onde 430 nm (Filtre Thorlab FB430-10).

Les figures 10A et 11A correspondent à deux parties d'une image de détection $I_d$, s'étendant respectivement autour de spermatozoïdes référencés 13 et 40. Les figures 10B et 11B correspondent respectivement aux $ROI'_p$, avec p=13 et p = 40 extraites de l'image acquise par le capteur d'image dans la bande spectrale verte. Les figures 10D et 11D correspondent respectivement aux $ROI'_p$, avec p=13 et p = 40 extraites de l'image acquise par le capteur d'image dans la bande spectrale rouge. Les figures 10C et 11C correspondent respectivement aux images de fluorescence intégrées à partir de N = 10 régions d'intérêt annotées $ROI'_p$, avec p=13 et p = 40 extraites l'image acquise par le capteur d'image dans la bande spectrale verte. Les figures 10E et 11E correspondent respectivement aux images de fluorescence intégrées à partir de N = 10 régions d'intérêt annotées $ROI'_p$, avec p=13 et p = 40 extraites de l'image acquise par le capteur d'image dans la bande spectrale rouge.

Les images de fluorescence, prises isolément les unes des autres (cf. figures 10B, 10D, 11B, 11D) ne permettent pas de conclure sur l'état des spermatozoïdes référencés 13 et 40. Les images de fluorescence intégrées (cf. figures 10C, 10E, 11C, 11E) permettent de conclure que les spermatozoïdes référencés 13 et 40 sont respectivement vivant (cf. figure 10C) et mort (cf. figure 11D).

Bien que décrite en lien avec des spermatozoïdes, l'invention s'applique à d'autres particules, en particulier des particules biologiques. Il peut par exemple s'agir de cellules.

L'invention s'applique à la détection de particules fluorescentes, qu'il s'agisse d'une fluorescence exogène, i-e induite par le marquage avec un marqueur fluorescent, ou d'une fluorescence endogène, de type autofluorescence.

L'invention peut également s'appliquer à l'observation de la luminescence, en particulier la bioluminescence, par exemple la bioluminescence de cellules dans un milieu de culture. Dans ce cas, le recours à une lumière d'excitation n'est pas nécessaire, la bioluminescence résultant d'une réaction chimique. Ainsi, selon ce mode de réalisation, le dispositif peut ne pas comporter de source d'excitation 12.

Qu'il s'agisse de fluorescence ou de luminescence, la durée d'intégration, c'est-à-dire la durée selon laquelle on cumule les régions d'intérêt définies sur chaque image de fluorescence, peut être de quelques secondes à quelques dizaines de minutes, voire davantage. C'est d'ailleurs un avantage conféré par l'invention : l'intégration d'images de fluorescence ou de luminescence prenant en compte le déplacement des particules, elle peut être réalisée avec un grand nombre d'images, selon une durée longue.

**Revendications**

1. Procédé d'observation d'une émission de lumière de fluorescence ou de luminescence d'une particule mobile dans un échantillon (2), l'échantillon comportant différentes particules se déplaçant selon des trajectoires différentes, la particule émettant la lumière de fluorescence ou de luminescence dans une bande spectrale d'émission ($\Delta\lambda m$), le procédé comportant :

   - a) illumination de l'échantillon dans une bande spectrale de détection ($\Delta\lambda d$), et formation d'une image de détection ($I_d$) de l'échantillon, dans la bande spectrale de détection, la bande spectrale de détection étant différente de la bande spectrale d'émission ;
   - b) formation d'une image d'émission ($I_m$), de l'échantillon, dans la bande spectrale d'émission ($\Delta\lambda m$);
   - l'image de détection et l'image d'émission étant obtenues à partir d'une acquisition d'une image de l'échantillon par un capteur d'image (20), dans la bande spectrale de détection et dans la bande spectrale d'émission,
   - les étapes a) et b) étant réitérées;
   - le procédé comportant également les étapes suivantes :

      - c) à partir de chaque image de détection ($I_d$) résultant de chaque itération des étapes a) et b), mise en œuvre d'un algorithme de suivi de la trajectoire de la particule de façon à détecter la particule sur chaque image de détection successivement acquise et déterminer une région d'intérêt ($ROI_p$) autour de la particule ;
      - d) à partir de la région d'intérêt ($ROI_p$) résultant de chaque étape c), extraction d'une région d'intérêt ($ROI'_p$) dans chaque image d'émission de l'échantillon successivement acquise, la région d'intérêt ($ROI'_p$) extraite dans chaque image d'émission ($I_m$) correspondant à la même particule détectée dans chaque image de détection ($I_d$) successivement acquise;

- e) sommation des régions d'intérêt (ROI'$_p$) extraites dans chaque étape d), de façon à former une image d'émission intégrée de la particule, représentative de la fluorescence ou de la luminescence de la particule, l'image d'émission intégrée de la particule étant soit une image obtenue par l'intégration de plusieurs régions d'intérêt extraites de l'étape d), soit un nombre correspondant à un cumul des intensités des images de fluorescence successives, dans les régions extraites de l'étape d).

2. Procédé selon la revendication 1, dans lequel :

   - la particule émet une lumière de fluorescence dans la bande spectrale d'émission lorsqu'elle est illuminée dans une bande spectrale d'excitation ($\Delta\lambda$e);
   - l'étape b) comporte une illumination de l'échantillon dans la bande spectrale d'excitation ;
   - la bande spectrale de détection ($\Delta\lambda$d) est distante de la bande spectrale d'excitation ($\Delta\lambda$e)
   - le capteur d'image est couplé à un filtre, de façon à bloquer la bande spectrale d'excitation ;

3. Procédé selon l'une quelconque des revendications 1 ou 2, dans lequel les étapes a) et b) sont mises en œuvre simultanément.

4. Procédé selon la revendication 3, dans lequel les étapes c), d) et e) sont mises en œuvre au cours de chaque itération des étapes a) et b) ou à la suite des itérations des étapes a) et b).

5. Procédé selon l'une quelconque des revendications 1 ou 2, dans lequel au cours de chaque itération, les étapes a) et b) sont mises en œuvre successivement

6. Procédé selon la revendication 5, dans lequel :

   • au moins deux itérations des étapes a) et b) sont effectuées successivement, selon une itération de rang n et une itération de rang n+1, n étant un entier strictement positif ;
   • l'étape d), comporte :

      (i) une interpolation des positions de la particule dans les images de détection résultant des étapes a) des itérations de rang n et n+1, de façon à estimer une position interpolée de la particule lors de l'étape b) de l'itération de rang n ;
      (ii) une extraction de la région d'intérêt dans l'image d'émission (I$_m$) formée lors de l'itération de rang n à partir de la position interpolée estimée lors de la sous-étape (ii).

7. Procédé selon l'une quelconque des revendications précédentes, dans lequel :

   - le capteur d'image (20) est un capteur d'image couleur ;
   - dans l'étape a), l'image de détection (I$_d$) de l'échantillon est obtenue à partir d'une première composante spectrale d'une l'image acquise par le capteur d'image, la première composante spectrale correspondant à tout ou partie de la bande spectrale de détection ;
   - et/ou dans l'étape b), l'image d'émission (I$_m$) de l'échantillon est obtenue à partir d'une deuxième composante spectrale de l'image acquise par le capteur d'image, la deuxième composante spectrale correspondant à tout ou partie de la bande spectrale d'émission ;
   - de façon que la même image (I), acquise par le capteur d'image, permet de former l'image de détection et l'image d'émission de l'échantillon.

8. Procédé selon l'une quelconque des revendications 1 à 6, dans lequel :

   - le capteur d'image (20) comporte un premier capteur d'image élémentaire (21) et un deuxième capteur d'image élémentaire (22), ainsi qu'un séparateur, le séparateur étant configuré pour envoyer la lumière,

      • dans la bande spectrale de détection, vers le premier capteur d'image élémentaire ;
      • dans la bande spectrale d'émission, vers le deuxième capteur d'image élémentaire ;

   - lors de chaque étape a), l'image de détection, dans la bande spectrale de détection, est acquise par le premier capteur d'image élémentaire ;
   - lors de chaque étape b), l'image d'émission, dans la bande spectrale d'émission, est acquise par le deuxième capteur d'image élémentaire.

9. Procédé selon l'une quelconque des revendications précédentes, dans lequel l'étape e) comporte le calcul d'une moyenne des régions d'intérêt (ROI'$_p$) extraites dans chaque image d'émission de l'échantillon, correspondant à la même particule.

10. Procédé selon l'une quelconque des revendications précédentes, dans lequel :

   - le critère d'arrêt est un nombre d'itérations prédéterminé ;
   - ou l'étape e) étant mise en œuvre au cours de chaque itération des étapes a) et b), le critère d'arrêt est une obtention d'une image d'émission intégrée dans laquelle le rapport signal sur bruit dépasse un seuil prédéterminé.
- ou l'étape e) étant mise en œuvre au cours de chaque itération des étapes a) et b), le procédé comporte un affichage de l'image d'émission intégrée , les itérations étant arrêtées par un utilisateur.

11. Dispositif (1) d'observation d'un échantillon comportant une particule mobile, la particule étant susceptible d'émettre une lumière de fluorescence ou de luminescence dans une bande spectrale d'émission, le dispositif comportant :

> - une source de lumière (11) de détection, configurée pour illuminer l'échantillon dans une bande spectrale de détection, distante de la bande spectrale d'émission;
> - un capteur d'image (20), configuré pour acquérir une image de l'échantillon, dans la bande spectrale de détection et la bande spectrale d'émission ;
> - le dispositif étant configuré pour maintenir l'échantillon face au capteur d'image, selon un plan d'échantillon ;
> - un filtre d'émission (18), disposé entre le capteur d'image et le plan d'échantillon, le filtre d'émission étant configuré pour transmettre la lumière dans la bande spectrale de détection et dans la bande spectrale d'émission ;
> - une unité de traitement (30), configurée pour former, à partir de l'image acquise par le capteur d'image ;
>
> > • une image de détection de l'échantillon, dans la bande spectrale de détection ($\Delta\lambda d$);
> > • une image d'émission de l'échantillon, dans la bande spectrale d'émission ($\Delta\lambda m$);
>
> - le dispositif étant tel que l'unité de traitement est programmée pour mettre en œuvre les étapes a) à e) d'un procédé selon l'une quelconque des revendications précédentes.

12. Dispositif selon la revendication 11, comportant une source de lumière d'excitation (12), configurée pour illuminer l'échantillon dans une bande spectrale d'excitation ($\Delta\lambda e$), distante de la bande spectrale d'émission et de la bande spectrale de détection.

13. Dispositif selon la revendication 12, dans lequel la source de lumière de détection et la source de lumière d'excitation sont configurées pour être activées simultanément ou séquentiellement, selon un décalage temporel inférieur à 100 ms ou à 10 ms.

14. Dispositif selon l'une quelconque des revendications 11 à 13, dans lequel le capteur d'image est un capteur d'image couleur.

15. Dispositif selon l'une quelconque des revendications 11 à 13, dans lequel le capteur d'image (20) comporte un premier capteur d'image élémentaire (21) et un deuxième capteur d'image élémentaire (22), ainsi qu'un séparateur (23), le séparateur étant configuré pour envoyer la lumière,

> - dans la bande spectrale de détection, vers le premier capteur d'image élémentaire;
> - dans la bande spectrale d'émission, vers le deuxième capteur d'image élémentaire.

**Patentansprüche**

1. Verfahren zur Beobachtung einer Fluoreszenz- oder Lumineszenzlichtemission eines beweglichen Teilchens in einer Probe (2), wobei die Probe unterschiedliche Teilchen umfasst, die sich entlang unterschiedlicher Bahnen bewegen, wobei das Teilchen das Fluoreszenz- oder Lumineszenzlicht in einem Emissionsspektralband ($\Delta\lambda m$) emittiert, wobei das Verfahren Folgendes umfasst:

> - a) Beleuchten der Probe in einem Detektionsspektralband ($\Delta\lambda d$) und Bilden eines Detektionsbilds ($I_d$) der Probe in dem Detektionsspektralband, wobei sich das Detektionsspektralband von dem Emissionsspektralband unterscheidet;
> - b) Bilden eines Emissionsbilds ($I_m$) der Probe in dem Emissionsspektralband ($\Delta\lambda m$);
> - wobei das Detektionsbild und das Emissionsbild anhand einer Erfassung eines Bilds der Probe durch einen Bildsensor (20) in dem Detektionsspektralband und in dem Emissionsspektralband erhalten werden,
> - wobei die Schritte a) und b) wiederholt werden;
> - wobei das Verfahren ferner die folgenden Schritte umfasst:
>
> > - c) anhand jedes Detektionsbilds ($I_d$), das sich aus jeder Wiederholung der Schritte a) und b) ergibt, Durchführen eines Algorithmus zur Verfolgung der Bahn des Teilchens, um das Teilchen auf jedem sukzessiv erfassten Detektionsbild zu detektieren und einen Bereich von Interesse ($ROI_p$) um das Teilchen herum zu bestimmen;
> > - d) anhand des Bereichs von Interesse ($ROI_p$), der sich aus jedem Schritt c) ergibt, Extrahieren eines Bereichs von Interesse ($ROI'_p$) in jedem sukzessiv erfassten Emissionsbild der Probe, wobei der in jedem Emissionsbild ($I_m$) extrahierte Bereich von Interesse ($ROI'_p$) demselben Teilchen entspricht, das in jedem sukzessiv erfassten Detektionsbild ($I_d$) detektiert wurde;

- e) Summieren der in jedem Schritt d) extrahierten Bereiche von Interesse (ROI'$_p$), um ein integriertes Emissionsbild des Teilchens zu bilden, das für die Fluoreszenz oder die Lumineszenz des Teilchens repräsentativ ist, wobei das integrierte Emissionsbild des Teilchens entweder ein Bild ist, das durch die Integration mehrerer extrahierter Bereiche von Interesse des Schritts d) erhalten wird, oder eine Zahl ist, die einer Kumulierung der Intensitäten der sukzessiven Fluoreszenzbilder in den extrahierten Bereichen des Schritts d) entspricht.

2. Verfahren nach Anspruch 1, wobei:

- das Teilchen ein Fluoreszenzlicht in dem Emissionsspektralband emittiert, wenn es in einem Anregungsspektralband ($\Delta\lambda e$) beleuchtet wird;
- Schritt b) das Beleuchten der Probe in dem Anregungsspektralband umfasst;
- das Detektionsspektralband ($\Delta\lambda d$) von dem Anregungsspektralband ($\Delta\lambda e$) entfernt ist;
- der Bildsensor mit einem Filter gekoppelt ist, um das Anregungsspektralband zu blockieren.

3. Verfahren nach einem der Ansprüche 1 oder 2, wobei die Schritte a) und b) simultan durchgeführt werden.

4. Verfahren nach Anspruch 3, wobei die Schritte c), d) und e) während jeder Wiederholung der Schritte a) und b) oder im Anschluss an die Wiederholungen der Schritte a) und b) durchgeführt werden.

5. Verfahren nach einem der Ansprüche 1 oder 2, wobei die Schritte a) und b) während jeder Wiederholung sukzessiv durchgeführt werden.

6. Verfahren nach Anspruch 5, wobei:

• mindestens zwei Wiederholungen der Schritte a) und b) sukzessiv gemäß einer Wiederholung von Rang n und einer Wiederholung von Rang n+1 vorgenommen werden, wobei n eine strikt positive ganze Zahl ist;
• Schritt d) Folgendes umfasst:

(i) Interpolieren der Positionen des Teilchens in den Detektionsbildern, die sich aus den Schritten a) der Wiederholungen von Rang n und n+1 ergeben, um eine interpolierte Position des Teilchens während des Schritts b) der Wiederholung von Rang n zu ermitteln;
(ii) Extrahieren des Bereichs von Interesse in dem Emissionsbild (I$_m$), das während der

Wiederholung von Rang n gebildet wurde, anhand der während des Unterschritts (ii) ermittelten interpolierten Position.

7. Verfahren nach einem der vorhergehenden Ansprüche, wobei:

- der Bildsensor (20) ein Farbbildsensor ist;
- in Schritt a) das Detektionsbild (I$_d$) der Probe anhand einer ersten Spektralkomponente eines durch den Bildsensor erfassten Bilds erhalten wird, wobei die erste Spektralkomponente dem gesamten oder einem Teil des Detektionsspektralbands entspricht;
- und/oder in Schritt b) das Emissionsbild (I$_m$) der Probe anhand einer zweiten Spektralkomponente des durch den Bildsensor erfassten Bilds erhalten wird, wobei die zweite Spektralkomponente dem gesamten oder einem Teil des Emissionsspektralbands entspricht;
- sodass es mit ein und demselben Bild (I), das durch den Bildsensor erfasst wird, möglich ist, das Detektionsbild und das Emissionsbild der Probe zu bilden.

8. Verfahren nach einem der Ansprüche 1 bis 6, wobei:

- der Bildsensor (20) einen ersten Elementarbildsensor (21) und einen zweiten Elementarbildsensor (22) sowie einen Teiler umfasst, wobei der Teiler dazu konfiguriert ist, das Licht folgendermaßen auszusenden:

• in dem Detektionsspektralband zu dem ersten Elementarbildsensor;
• in dem Emissionsspektralband zu dem zweiten Elementarbildsensor;

- während jedes Schritts a) das Detektionsbild in dem Detektionsspektralband durch den ersten Elementarbildsensor erfasst wird;
- während jedes Schritts b) das Emissionsbild in dem Emissionsspektralband durch den zweiten Elementarbildsensor erfasst wird.

9. Verfahren nach einem der vorhergehenden Ansprüche, wobei Schritt e) das Berechnen eines Mittelwerts der in jedem Emissionsbild der Probe extrahierten Bereiche von Interesse (ROI'$_p$), die demselben Teilchen entsprechen, umfasst.

10. Verfahren nach einem der vorhergehenden Ansprüche, wobei:

- das Stoppkriterium eine vorbestimmte Anzahl von Wiederholungen ist;
- oder, wenn Schritt e) während jeder Wiederholung der Schritte a) und b) durchgeführt wird,

**12**

das Stoppkriterium das Erhalten eines integrierten Emissionsbilds ist, in dem das Signal-Rausch-Verhältnis einen vorbestimmten Schwellenwert überschreitet;
- oder, wenn Schritt e) während jeder Wiederholung der Schritte a) und b) durchgeführt wird, das Verfahren das Anzeigen des integrierten Emissionsbilds umfasst, wobei die Wiederholungen durch einen Benutzer gestoppt werden.

11. Vorrichtung (1) zur Beobachtung einer Probe, die ein bewegliches Teilchen umfasst, wobei das Teilchen zur Emission eines Fluoreszenz- oder Lumineszenzlichts in einem Emissionsspektralband fähig ist, wobei die Vorrichtung Folgendes umfasst:

    - eine Detektionslichtquelle (11), die dazu konfiguriert ist, die Probe in einem Detektionsspektralband, das von dem Emissionsspektralband entfernt ist, zu beleuchten;
    - einen Bildsensor (20), der dazu konfiguriert ist, ein Bild der Probe in dem Detektionsspektralband und dem Emissionsspektralband zu erfassen;
    - wobei die Vorrichtung dazu konfiguriert ist, die Probe gemäß einer Probenebene gegenüber dem Bildsensor zu platzieren;
    - einen Emissionsfilter (18), der zwischen dem Bildsensor und der Probenebene angeordnet ist, wobei der Emissionsfilter dazu konfiguriert ist, das Licht in dem Detektionsspektralband und in dem Emissionsspektralband zu übertragen;
    - eine Verarbeitungseinheit (30), die dazu konfiguriert ist, anhand des durch den Bildsensor erfassten Bilds Folgendes zu bilden:

        • ein Detektionsbild der Probe in dem Detektionsspektralband ($\Delta\lambda d$);
        • ein Emissionsbild der Probe in dem Emissionsspektralband ($\Delta\lambda m$);

    - wobei die Vorrichtung derart ist, dass die Verarbeitungseinheit dazu programmiert ist, die Schritte a) bis e) eines Verfahrens nach einem der vorhergehenden Ansprüche durchzuführen.

12. Vorrichtung nach Anspruch 11, die eine Anregungslichtquelle (12) umfasst, die dazu konfiguriert ist, die Probe in einem Anregungsspektralband ($\Delta\lambda e$), das von dem Emissionsspektralband und dem Detektionsspektralband entfernt ist, zu beleuchten.

13. Vorrichtung nach Anspruch 12, wobei die Detektionslichtquelle und die Anregungslichtquelle dazu konfiguriert sind, simultan oder sequenziell gemäß einem Zeitversatz von weniger als 100 ms oder 10 ms aktiviert zu werden.

14. Vorrichtung nach einem der Ansprüche 11 bis 13, wobei der Bildsensor ein Farbbildsensor ist.

15. Vorrichtung nach einem der Ansprüche 11 bis 13, wobei der Bildsensor (20) einen ersten Elementarbildsensor (21) und einen zweiten Elementarbildsensor (22) sowie einen Teiler (23) umfasst, wobei der Teiler dazu konfiguriert ist, das Licht folgendermaßen auszusenden:

    - in dem Detektionsspektralband zu dem ersten Elementarbildsensor;
    - in dem Emissionsspektralband zu dem zweiten Elementarbildsensor.

## Claims

1. Method for observing an emission of fluorescence light or luminescence light from a moving particle in a sample (2), the sample comprising particles moving in different directions, the particle emitting the fluorescence or luminescence light in a spectral emission band ($\Delta\lambda m$), the method comprising:

    - a) illuminating the sample in a spectral detection band ($\Delta\lambda d$), and forming a detection image ($I_d$) of the sample in the spectral detection band, the spectral detection band being different from the spectral emission band;
    - b) forming an emission image ($I_m$) of the sample in the spectral emission band ($\Delta\lambda m$);
    - the detection image and the emission image being obtained on the basis of an acquisition of an image of the sample by an image sensor (20), in the spectral detection band and in the spectral emission band,

    the method being **characterized in that**:

    - steps a) and b) are reiterated;
    - the method also comprises the following steps:

        - c) on the basis of each detection image ($I_d$) resulting from each iteration of steps a) and b), executing a tracking algorithm to detect the particle on each detection image successively acquired and determining a region of interest ($ROI_p$) around the particle;
        - d) on the basis of the region of interest ($ROI_p$) resulting from each step c), extracting a region of interest ($ROI'_p$) from each emission image of the sample successively acquired, the region of interest ($ROI'_p$) extracted from each emission image ($I_m$) corresponding to the same particle detected in each successively acquired detection image ($I_d$);

- e) summing the regions of interest (ROI_p) extracted in each step d) so as to form an integrated emission image of the particle, representative of the fluorescence or luminescence of the particle, wherein the integrated emission image of the particle is either an image obtained by the cumulation of many regions of interest extracted from step d), or a number corresponding to a cumulation of intensities of the successive fluorescence images, in the regions of interest extracted from step d)

2. Method according to Claim 1, wherein:

- the particle emits a fluorescence light in the spectral emission band when it is illuminated in a spectral excitation band ($\Delta\lambda$e);
- step b) comprises illuminating the sample in the spectral excitation band ;
- the spectral detection band ($\Delta\lambda$d) is remote from the spectral excitation band ($\Delta\lambda$e);
- the image sensor is coupled to a filter so as to block the spectral excitation band.

3. Method according to any one of claims 1 or 2, wherein steps a) and b) are executed simultaneously.

4. Method according to Claim 3, wherein steps c), d) and e) are executed in each iteration of steps a) and b) or following the iterations of steps a) and b).

5. Method according to any one of Claim 1 or 2, wherein, in each iteration, steps a) and b) are executed simultaneously.

6. Method according to Claim 5, wherein:

• at least two iterations of steps a) and b) are executed successively, according to an iteration of rank n and an iteration of rank n+1, n being a strictly positive integer;
• step d) comprises:

(i) interpolating the positions of the particle in the detection images resulting from steps a) of the iterations of rank n and n+1, so as to estimate an interpolated position of the particle in step b) of the iteration of rank n;
(ii) extracting the region of interest from the emission image (I_m) formed in the iteration of rank n on the basis of the interpolated position estimated in sub-step (ii).

7. Method according to any of the preceding claims, wherein:

- the image sensor (20) is a colour image sensor;

- in step a), the detection image (I_d) of the sample is obtained on the basis of a first spectral component of the image acquired by the image sensor, the first spectral component corresponding to all or part of the spectral detection band;
- and/or, in step b), the emission image (I_m) of the sample is obtained on the basis of a second spectral component of the image acquired by the image sensor, the second spectral component corresponding to all or part of the spectral emission band;
- in such a way that the same image (I) acquired by the image sensor may be used to form the detection image and the emission image of the sample.

8. Method according to any of claims 1 to 6, wherein:

- the image sensor (20) comprises a first elementary image sensor (21) and a second elementary image sensor (22), together with a beam splitter, the beam splitter being configured to send light,

• in the spectral detection band, towards the first elementary image sensor;
• and in the spectral emission band, towards the second elementary image sensor;

- In each step a), the detection image, in the spectral detection band, is acquired by the first elementary image sensor;
- in each step b), the emission image, in the spectral emission band, is acquired by the second elementary image sensor.

9. Method according to any of the preceding claims, wherein step e) comprises calculating an average of the regions of interest (ROI'_p), extracted from each emission image of the sample, corresponding to the same particle.

10. Method according to any of the preceding claims, wherein:

- the stop criterion is a predetermined number of steps;
- or, step e) being executed in each iteration of steps a) and b), the stop criterion is the obtaining of an integrated emission image in which the signal-to-noise ratio exceeds a predetermined threshold,
- or, step e) being executed in each iteration of steps a) and b), the method comprises a display of the integrated emission image, the iterations being stopped by a user.

11. Device f(1) or observing a sample comprising a moving particle, the particle being capable of emitting a fluorescence light or a luminescence light in a spectral emission band, the device comprising:

- a detection light source (11), configured to illuminate the sample in a spectral detection band, remote from the spectral emission band;
- an image sensor (20), configured to acquire an image of the sample, in the spectral detection band and the spectral emission band;
- the device being configured to keep the sample facing the image sensor on a sample plane;
- an emission filter (18), placed between the image sensor and the sample plane, the emission filter being configured to transmit light in the spectral detection band and in the spectral emission band;
- a processing unit (30) configured to form, on the basis of the image acquired by the image sensor:

  • a detection image of the sample in the spectral detection band ($\Delta\lambda d$);
  • an emission image of the sample, in the spectral emission band ($\Delta\lambda m$);

- the device being such that the processing unit is programmed to execute steps a) to e) of a method according to Claim 1.

12. Device according to Claim 11, comprising an excitation light source (12) configured to illuminate the sample in a spectral excitation band ($\Delta\lambda e$), remote from the spectral emission band and the spectral detection band.

13. Device according to Claim 12, wherein the detection light source and the excitation light source are configured to be activated simultaneously or sequentially, with a time shift of less than 100 ms or 10 ms.

14. Device according to any one of claims 11 to 13, wherein the image sensor is a colour image sensor.

15. Device according to any one of claims 11 to 13, wherein the image sensor (20) comprises a first elementary image sensor (21) and a second elementary image sensor (22), together with a beam splitter (23), the beam splitter being configured to send light,

- in the spectral detection band, towards the first elementary image sensor;
- and in the spectral emission band, towards the second elementary image sensor.

**Fig. 1**

**Fig. 2A**

**Fig. 2B**

**Fig. 3**

**Fig. 4A**

**Fig. 4B**

```
┌─────────┐     ┌─────────┐
│   100   │     │   110   │
└─────────┘     └─────────┘
        │   │
     I_d │   │ I_m
        ▼   ▼
    ┌─────────┐
    │   120   │
    └─────────┘
         │
      ROI_p
         ▼
    ┌─────────┐
    │   130   │
    └─────────┘
         │
      ROI'_p
         ▼
    ┌─────────┐
    │   140   │
    └─────────┘
         │ I_p,N
```

**Fig. 5A**

```
┌─────────┐     ┌─────────┐
│   100   │     │   110   │
└─────────┘     └─────────┘
        │   │
     I_d │   │ I_m
        ▼   ▼
    ┌─────────┐
    │   120   │
    └─────────┘
         │
      ROI_p
         ▼
    ┌─────────┐
    │   130   │
    └─────────┘
         │
      ROI'_p
         ▼
    ┌─────────┐
    │   140   │
    └─────────┘
         │ I_p,N
         ▼
```

**Fig. 5B**

```
    ┌─────────┐
    │   100   │
    └─────────┘
         │
       I_d
         ▼
    ┌─────────┐
    │   110   │
    └─────────┘
         │
       I_m
         ▼
    ┌─────────┐
    │   120   │
    └─────────┘
         │
      ROI_p
         ▼
    ┌─────────┐
    │   125   │
    └─────────┘
         │
         ▼
    ┌─────────┐
    │   130   │
    └─────────┘
         │
      ROI'_p
         ▼
    ┌─────────┐   I_p,N
    │   140   │ ─────────▶
    └─────────┘
```

**Fig. 5C**

**Fig. 6A**

**Fig. 6B**

**Fig. 7**

Fig. 8A

Fig. 8B

Fig. 8C

Fig. 8D

Fig. 9A

Fig. 9B

Fig. 10A

Fig. 10B

Fig. 10C

Fig. 10D

Fig. 10E

Fig. 11A

Fig. 11B

Fig. 11C

Fig. 11D

Fig. 11E

## RÉFÉRENCES CITÉES DANS LA DESCRIPTION

**Documents brevets cités dans la description**

- US 20180293426 A
- WO 2017214023 A
- US 2016169801 A
- US 6259807 B